(19) Europäisches Patentamt / European Patent Office / Office européen des brevets

(11) **EP 4 662 198 B1**

(12) **EUROPEAN PATENT SPECIFICATION**

(45) Date of publication and mention
of the grant of the patent:
**29.07.2026 Bulletin 2026/31**

(21) Application number: **24702356.7**

(22) Date of filing: **29.01.2024**

(51) International Patent Classification (IPC):
***C07C 277/08*** (2006.01)   ***C07C 279/14*** (2006.01)
***A23K 40/10*** (2016.01)   ***C12P 13/04*** (2006.01)

(52) Cooperative Patent Classification (CPC):
(C-Sets available)
**C07C 277/08; C12P 13/04; A23K 20/142;**
A23K 40/10   (Cont.)

(86) International application number:
**PCT/EP2024/052013**

(87) International publication number:
**WO 2024/165344 (15.08.2024 Gazette 2024/33)**

(54) **PROCESS FOR PRODUCING FREE-FLOWING PARTICLES COMPRISING OR CONSISTING OF AN N-GUANYLAMINO ACID**

VERFAHREN ZUR HERSTELLUNG VON FLIESSFÄHIGEN PARTIKELN MIT ODER AUS EINER N-GUANYLAMINOSÄURE

PROCÉDÉ DE PRODUCTION DE PARTICULES À ÉCOULEMENT LIBRE COMPRENANT OU CONSTITUÉES D'UN ACIDE N-GUANYLAMINO

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**

(30) Priority: **06.02.2023 EP 23155155**

(43) Date of publication of application:
**17.12.2025 Bulletin 2025/51**

(73) Proprietor: **Evonik Operations GmbH
45128 Essen (DE)**

(72) Inventors:
• **HEINING, Martin
63791 Karlstein am Main (DE)**
• **LEBERT, Jochen
63864 Glattbach (DE)**
• **STAHL, Timo
Kuala Lumpur, 50480 (MY)**
• **GRIMMER, Ralf
63579 Freigericht (DE)**
• **HEIN, Lukas
63755 Alzenau (DE)**
• **MAYER, Alexander
82452 Planegg (DE)**
• **MERZ, Juliane
44139 Dortmund (DE)**

(74) Representative: **Evonik Patent Association
c/o Evonik Industries AG
IP Management
Postcode 84/339
Rodenbacher Chaussee 4
63457 Hanau (DE)**

(56) References cited:
EP-A1- 3 839 051   CN-A- 113 651 726
US-A1- 2010 143 703   US-A1- 2022 388 948

(52) Cooperative Patent Classification (CPC): (Cont.)

C-Sets
**C07C 277/08, C07C 279/14**

**EP 4 662 198 B1**

**Description**

The present invention relates to a process for producing free-flowing particles comprising or consisting of an *N*-guanylamino acid.

*N*-guanylamino acids are derivatives of amino acids with a guanidine group, which are obtainable by addition of cyanamide to the amino acids in question. The most important *N*-guanylamino acid is guanidinoacetic acid, also known as *N*-guanylglycine. It is an endogenous substance in animals and humans which takes a central role in the biosynthesis of creatine. Creatine can be taken by the diet, and it can also be formed endogenously. Its biosynthesis proceeds from glycine and L-arginine. Guanidinoacetic acid is formed in the mammalian organism, primarily in the kidneys, by transferring the guanidine group of L-arginine by the enzyme L-Arg:Gly-amidinotransferase (AGAT) to the amino acid glycine. From L-arginine, L-ornithine is thus produced, which is metabolized in the urea cycle by carbamoylation to L-citrulline. In a further step, guanidinoacetic acid is methylated to creatine with S-adenosyl methionine by the enzyme guanidinoacetate *N*-methyltransferase (GAMT). The thus obtained creatine is released into the bloodstream of the mammal and transported to the target organs through the cell membrane into the cells, which takes place via a specific creatine transporter.

Guanidinoacetic acid was first prepared in 1861 by Adolph Strecker by addition of cyanamide to glycine in aqueous solution, specifically in weak ammoniacal aqueous solution (M. Strecker, compt. Rend. 1861, 52, 1212; cited in Ber. Chem. Ges. (now Eur. J. Inorg. Chem.) 1904, 41, 4385). In later publications, guanidinoacetic acid was prepared from cyanamide and glycine in iso-propanol as solvent in the presence of sodium hydroxide or sodium carbonate as base (CN 102329250 A or CN 101462983 A). Since iso-propanol easily forms flammable mixtures of vapor and air, it is not a good and advisable solvent in an industrial process. However, the synthesis of guanidinoacetic acid from cyanamide and glycine in aqueous alkaline solution under the reaction conditions disclosed in the prior art is also not free from any problems. Specifically, the particles obtained from said synthesis have strong limitations for their commercial use. For example, a significant amount of these particles is too fine, or is not abrasion resistant. Both aspects also cause dust problems during the handling of the particles. In addition, these particles also have unsatisfying flow characteristics.

The physical properties of the thus obtained particles can be improved by granulation (see WO 2009/012960 A) or coating (see CN 110663821 A). However, the thus obtained N-guanylamino acid comprising particles always contain an auxiliary substance, which reduces product purity and quality. And even more the additional processing makes the product more expensive.

The published patent application US 2022/388948 A1 discloses the preparation of a particular crystal form ("form B") by crystallizing guanidinoacetic acid in the presence of another guanidine compound. The form B is described in this document as a free-flowing, granular product.

The fermentative production of N-guanylamino acid is faced with similar problems. Products obtained from fermentation processes may contain biomass and other impurities. This, however, impairs the product purity and quality.

Accordingly, there was still a need for an improvement of the physical properties of particles comprising or consisting of an N-guanylamino acid, e.g., guanidinoacetic acid (GAA). It was found that this problem is solved in that a solution or suspension comprising an N-guanylamino acid, e.g., guanidinoacetic acid, is subjected first to a pH shift to a pH below the pKa value of the N-guanylamino acid, followed by another pH shift back to a pH larger than the pKa value of the N-guanylamino acid and lower than the pKb value of the N-guanylamino acid.

One object of the present invention is therefore a process for the preparation of free-flowing particles comprising or consisting of an N-guanylamino acid, e.g., guanidinoacetic acid, wherein the process comprises the steps of

a) providing an N-guanylamino acid, e.g., GAA, comprising solution or suspension,
b) shifting the pH of the solution or suspension provided in step a) to a pH lower than the pKa value of the N-guanylamino acid, e.g., GAA, and
c) shifting the pH of the solution or suspension obtained in step b) to a pH larger than the pKa value of the N-guanylamino acid and lower than the pKb value of the N-guanylamino acid, e.g., GAA, wherein the step c) further comprises the precipitation or crystallization of the particles comprising or consisting of the N-guanylamino acid, e.g., guanidinoacetic acid,

wherein the pH in any of the steps b) and c) is measured by means of a pH electrode.

On the basis of guanidinoacetic acid as an example for an N-guanylamino acid, the experimental data show that the process according to the present invention always leads to particles with a better pourability and thus improved flow characteristics: The guanidinoacetic acid particles obtained by the process according to the present invention always have improved flow characteristics, in particular, a better pourability than the commercially available guanidinoacetic acid and granulated guanidinoacetic acid. These results are independent from the preceding production of the guanidinoacetic acid, i.e., whether it was prepared chemically or fermentatively.

It is believed that the pH shift performed in step b) of the process according to the present invention leads to a protonation of a nitrogen atom of the guanidine group of the N-guanylamino acid, e.g., guanidinoacetic acid. This facilitates the solvation or dissolution of the N-guanylamino acid. Thus, a renewed precipitation or crystallization is avoided and the dissolved *N*-guanylamino acid is removed from the precipitation/dissolution equilibrium - in contrast to the processes of the prior art.

It is believed that the pH shift in step c) induces the precipitation or crystallization of the *N*-guanylamino acid, e.g., guanidinoacetic acid, from the solution or suspension. The thus precipitated or crystallized *N*-guanylamino acid, e.g., guanidinoacetic acid, is then available for any further processing or use. For this purpose, the precipitated or crystallized *N*-guanylamino acid, e.g., guanidinoacetic acid, is separated from the solution or suspension and dried. However, it is also possible to further process the precipitated or crystallized *N*-guanylamino acid, e.g., guanidinoacetic acid, directly in the liquid medium, for example to prepare a dispersion comprising the N-guanylamino acid.

According to the present invention the step c) further comprises the precipitation or crystallization of the particles comprising or consisting of the *N*-guanylamino acid, e.g., guanidinoacetic acid.

In an embodiment the process according to the present invention further comprises the steps of

d) separating the precipitated or crystallized particles comprising or consisting of N-guanylamino acid, e.g., guanidinoacetic acid, from the solution or suspension obtained in step c), and
e) drying the particles separated in step d).

In step e) the particles are dried to dryness. The temperatures and the time applied for step e) depend on the solvent of the solution or suspension provided in step a) of the process according to the present invention.

First, the solvent of the solution or suspension provided in step a) is given by the preceding process resulting in the formation of the N-guanylamino acid, e.g., guanidinoacetic acid. When the N-guanylamino acid was formed in a chemical reaction, the solvent is preferably water, an organic solvent, for example iso-propanol, or a mixture of water with an organic solvent. In the context of the present invention suitable organic solvents are water-miscible organic solvents, in particular polar and/or protic organic solvents. When the *N*-guanylamino acid was formed in a fermentation process, the solvent is typically water. In any case, it is preferred that the medium of the solution or suspension comprises or consists of a protic solvent, e.g., an alcohol or water, which facilitates the protonation of the guanidine group of the *N*-guanylamino acid. In addition to the aforementioned solvents the solution of suspension may further comprise additional solubilizing agents.

In one embodiment of the process according to the present invention the solvent of the solution or suspension provided in step a) comprises or consists of water, an organic solvent, or a mixture thereof.

Preferably, the solvent of the solution or suspension provided in step a) is water.

In order to get the *N*-guanylamino acid dissolved as complete and as fast as possible, it is beneficial to protonate the guanidine group of the N-guanylamino acid as complete as possible. This is achieved when the pH of the solution or suspension is shifted in step b) to a pH lower than the pKa value of the *N*-guanylamino acid. It is beneficial to hold the thus set pH value for a certain time since this also contributes to get the guanidine group of the *N*-guanylamino acid protonated as complete as possible.

In order to perform the following pH shift in step c) as complete and as fast as possible, it is beneficial to deprotonate the guanidine group of the *N*-guanylamino acid as complete as possible. This is achieved in step c) when the pH of the solution or suspension obtained in step b) is shifted to a pH larger than the pKa value of the *N*-guanylamino acid and lower than the pKb value of the *N*-guanylamino acid. It is beneficial to hold the thus set pH value for a certain time since this also contributes to get the guanidine group of the *N*-guanylamino acid deprotonated as complete as possible.

The process according to the present invention is not subject to any limitation regarding the time for which the individual pH value set in step b) and/or step c) is held, provided that said time is sufficient to achieve the effect(s) of the step(s) in question. For example, the time for which the pH value is held in any step b) and/or c) can be up to 30 minutes, from 5 to 25 minutes, or from 10 to 20 minutes.

In another embodiment of the process according to the present invention the pH value(s) set in step b) and/or c) is/are held for a time of up to 30 minutes.

In principle, the process according to the present invention is not subject to any limitations regarding a specific *N*-guanylamino acid. However, since guanidinoacetic acid is the commercially most relevant *N*-guanylamino acid, it is preferred that the *N*-guanylamino acid is guanidinoacetic acid.

In an embodiment of the process according to the present invention the N-guanylamino acid is guanidinoacetic acid.

The pH of the solution or suspension provided in step a) depends on the individual N-guanylamino acid, e.g., guanidinoacetic acid, comprised in said solution or suspension and on the preceding synthesis for preparation of the *N*-guanylamino acid in question, e.g., guanidinoacetic acid. For example, when the *N*-guanylamino acid, e.g., guanidinoacetic acid, was prepared in a chemical synthesis, the pH of the solution or suspension provided in step a) is preferably in the neutral or alkaline range, i.e., the pH is larger than the pKa and lower than the pKb of the *N*-guanylamino acid, e.g., guanidinoacetic acid. It is preferred that in this case the pH ranges from ca. 7 to a value lower than the pKb of the *N*-

guanylamino acid, e.g., from ca. 7 to a value lower than ca. 11. When the *N*-guanylamino acid, e.g., guanidinoacetic acid, was prepared in a biochemical synthesis, i.e., in a fermentation, the pH of the solution or suspension provided in step a) is preferably in the neutral range, i.e., the pH is larger than the pKa and lower than the pKb of the *N*-guanylamino acid, e.g., guanidinoacetic acid. It is preferred that in this case the pH ranges from ca. 5 to ca. 9, or from ca. 6 to ca. 8. Thus, in any case the pH of the solution or suspension provided in step a) is always larger than the pKa and lower than the pKb of the N-guanylamino acid, e.g., guanidinoacetic acid. Further, the solvent or solvent mixture of the solution or suspension provided in step a) may also have an effect on the pH.

In the context of the present invention, any specific values mentioned for pH values always refer to the respective values measured in an aqueous solution or suspension using a pH electrode. However, the specific value of the pH in any of the steps a) to c) may be influenced by the solvent or solvent mixture of the solution or suspension provided in step a), the individual N-guanylamino acid, e.g., guanidinoacetic acid, comprised in said solution or suspension and/or the preceding synthesis for preparation of the *N*-guanylamino acid, in question, e.g., guanidinoacetic acid. Therefore, the process according to the present invention is not subject to any limitation regarding any specific pH values in any of the steps a), b) and/or c), provided that they achieve the beneficial effects of the present invention. Rather, any deviations from the values explicitly mentioned above and below are still encompassed by the scope of the present invention, provided that they allow to achieve the benefits of the present invention.

For example, when the *N*-guanylamino acid comprised in the solution or suspension provided in step a) is guanidinoacetic acid, the pH of said solution or suspension is shifted in step b) to a value lower than the pKa of guanidinoacetic acid, i.e., lower than approximately 2.9, measured by a pH-electrode, followed by shifting the pH in step c) to a value larger than the pKa of guanidinoacetic acid, i.e., larger than approximately 2.9, measured by a pH-electrode, and lower than the pKb of guanidinoacetic acid, i.e., lower than approximately 10.91. Regarding step c), it is further preferred that the pH is set to a value, at which the crystallization of the N-guanylamino acid, e.g., guanidinoacetic acid, is initiated, for example to a value in the range of from approximately 5 to lower than the pKb of guanidinoacetic acid, i.e., lower than approximately 10.91.

In a preferred embodiment of the process according to the present invention, when the N-guanylamino acid comprised in the solution or suspension provided in step a) is guanidinoacetic acid, the pH of the solution or suspension obtained in step b) is lower than the pKa of guanidinoacetic acid. Preferably, in that case the pH is lower than approximately 2.9.

In another preferred embodiment of the process according to the present invention, when the N-guanylamino acid comprised in the solution or suspension provided in step a) is guanidinoacetic acid, the pH of the solution or suspension obtained in step c) is larger than the pKa of guanidinoacetic acid and lower than the pKb of guanidinoacetic acid. Preferably, in said case the pH is larger than approximately 2.9 and lower than the pKb of guanidinoacetic acid, in particular, the pH is larger than 4 and lower than approximately 10.91 or is between 5 and lower than approximately 10.91. In particular, the crystallization of guanidinoacetic acid is initiated at a pH of 5 or more.

The process according to the present invention is also not subject to any specific temperature in any of the steps a), b) and/or c), provided that the chosen temperature does not have any negative effect on the results of any of these steps. In principle, the temperature in any of the steps a) to c) of the process according to the present invention is given by the solvent or solvent mixture of the solution or suspension, and the solubility of the *N*-guanylamino acid in question, e.g., guanidinoacetic acid, in the said solvent or solvent mixture. The temperature should be chosen as to allow the best possible solubility of the *N*-guanylamino acid in question, e.g., guanidinoacetic acid, without negative side effects. For example, that ammonia is not released and does not bubble out of the solution or suspension when ammonia or ammonium hydroxide is used as base in step b). Generally speaking, it is preferred that the temperature in any of steps a) to c), in particular in step b) and/or c), is less than 100 °C, preferably 95 or 90 °C at the most, in particular 80 or 85 °C at the most, e.g., the temperature ranges from 20 °C to 95 °C, from 20 °C to 90 °C, from 20 °C to 85 °C or from at 20 °C to 80 °C.

In principle, the process according to the present invention is not subject to any limitations regarding the use of a specific acid in step b) and/or a specific base in step c), provided that the strength of the acid and/or base is suitable to shift the pH of the solution or suspension to the required pH ranges. Therefore, it is possible to use any inorganic or organic acid or mixture thereof, which meets this proviso, and any inorganic or organic base or mixture thereof, which meets this proviso, in step b) and/or step c). For example, sulphuric acid can be used in step b), and/or caustic soda (NaOH) can be used in step c).

In principle, the process according to the present invention is also not subject to any limitations regarding the origin of the N-guanylamino acid, e.g., guanidinoacetic acid, or of the *N*-guanylamino acid, e.g., guanidinoacetic acid, comprising solution or suspension. Therefore, the *N*-guanylamino acid, e.g., guanidinoacetic acid, used in step a) or the *N*-guanylamino acid, e.g., guanidinoacetic acid, comprising solution or suspension provided in step a) can result from a chemical or fermentative process.

In one embodiment of the process according to the present invention the *N*-guanylamino acid, e.g., guanidinoacetic acid, comprising solution or the suspension provided in step a) or the *N*-guanylamino acid, e.g., guanidinoacetic acid, contained in said solution or suspension results from the chemical reaction of an amino acid with cyanamide.

In another embodiment of the process according to the present invention the *N*-guanylamino acid, e.g., guanidinoacetic acid, comprising solution or the suspension provided in step a) or the *N*-guanylamino acid, e.g., guanidinoacetic acid, contained in said solution or suspension results from the fermentative production of the *N*-guanylamino acid, e.g., guanidinoacetic acid.

The process according to the present invention allows to provide particles comprising or consisting of an *N*-guanylamino acid, e.g., guanidinoacetic acid, with improved, superior handling properties, compared to the *N*-guanylamino acid, e.g., guanidinoacetic acid, comprising particles of the prior art. Therefore, the process according to the present invention does not require any granulation step, in contrast to the processes of the prior art.

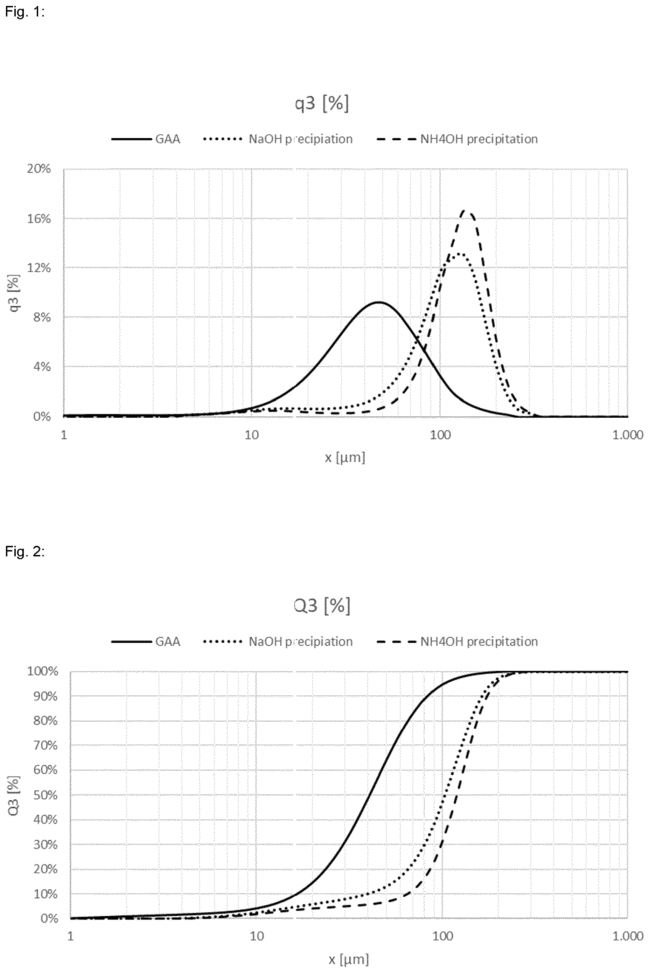

It was found that the particles obtained by the process according to the present invention, comprising or consisting of an *N*-guanylamino acid, e.g., guanidinoacetic acid, are large uniform particles. This is also illustrated in Figures 1 and 2 by way of example for guanidinoacetic acid. Figure 1 shows a comparison of the sum distribution for the particle size of the granulated guanidinoacetic acid (GAA) particles not according to the invention (black, full line) with the ungranulated GAA particles precipitated with NaOH according to the invention (black dotted line) and GAA particles precipitated with $NH_4OH$ according to the invention (black, broken line). Figure 2 shows a comparison of the density distribution for the particle size of the granulated GAA particles not according to the invention (black, full line) with the ungranulated GAA particles precipitated with NaOH according to the invention (black dotted line) and GAA particles precipitated with $NH_4OH$ according to the invention (black, broken line). These Figures show that the particles obtained by the process according to the present invention have larger particle size diameters than granulated GAA particles and/or the GAA subjected to step a) of the process according to the present invention. It is believed that the larger particle size diameter of the particles obtained by the process according to the present invention leads to their improved flow characteristics, e.g., improved pourability.

**Description of the Figures:**

Fig. 1: Comparison of the sum distribution for the particle size of the GAA particles, granulated not according to the invention (black, full line) with the GAA particles, ungranulated NaOH precipitated according to the invention (black dotted line) and GAA particles, $NH_4OH$ precipitated according to the invention (black, broken line)

Fig. 2: Comparison of the density distribution for the particle size of the GAA particles, granulated not according to the invention (black, full line) with the GAA particles, ungranulated NaOH precipitated according to the invention (black dotted line) and GAA particles, $NH_4OH$ precipitated according to the invention (black, broken line)

Fig. 3: Results for the determination of the angle of response of the tested products.

Fig. 4: Schematic representation of the pourability test.

Fig. 5: Results for the determination of the pourability of the tested products.

**Examples:**

1. Examples according to the invention

1.1 GAA ungranulated, NaOH precipitated

A glass beaker was filled with 100.3 g of guanidinoacetic acid (GAA) and water was added to a total weight of 1002.5 g. The thus obtained suspension was agitated from the top with an impeller (Minister 20 digital) with 150 rounds per minute. The suspension had a temperature of 20.9 °C and the pH of the suspension was 9.38. Sulphuric acid (80% w/w) was added to this suspension over a period of 17 minutes. After addition of 73.3 g of the sulphuric acid, the suspension had a pH value of 1.02 and clarified. Next, a total of 266.2 g of caustic soda (20 % w/w) was added over a period of 17 minutes to reach a pH value of 8.3, which led to a temperature increase to 37.8 °C. A precipitate was formed, which was separated from the solution via a suction filter and dried overnight at a temperature of 60 °C.

1.2 GAA ungranulated, $NH_4OH$ precipitated

A glass beaker was filled with 100.1 g of guanidinoacetic acid (GAA) and water was added to a total weight of 1002.5 g. The thus obtained suspension was agitated from the top with an impeller (Minister 20 digital) with 150 rounds per minute. The suspension had a temperature of 20.2 °C and the pH of the suspension was 9.44. Sulphuric acid (80% w/w) was added to this suspension over a period of 17 minutes. After addition of 71.5 g of the sulphuric acid, the suspension had a pH value of 1.02 and clarified. Next, a total of 85.4 g ammonia water (25.5 w/w) was added over a period of 20 minutes to reach a pH value of 8.01, which led to a temperature increase to 34.8 °C. A precipitate was formed, which was separated from the solution via a suction filter and dried overnight.

1.3 GAA ungranulated, from fermentation

A guanidinoacetic acid comprising fermentation broth with a pH value of 7.0 was titrated under stirring to a pH of 1.5 at 50 °C using sulphuric acid (75 % w/w). After dissolution of GAA, the biomass was removed via ultrafiltration (UF: ATECH Al2O3 tube model, 20 kDa cut of) at 50 °C and concentrated (retentate). The resulting retentate was diafiltered (DF) twice with acidic water (pH 1 adjusted with sulphuric acid (75 % w/w)). The (UF/DF) permeate was concentrated twice in a rotary evaporator (80 °C and 250 mbar). The thus obtained concentrated permeate was mixed with ammonia water (25 % w/w) at 50 °C to a final pH of 7.2. The resulting precipitate was separated from the solution and washed with water (2x solid volume) using a suction filter and dried overnight at 60 °C.

2. Examples not according to the invention

2.1 CreAmino®

Commercial guanidinoacetic acid (CreAmino®) was purchased from AlzChem, Germany.

2.2 Chemically produced GAA dried,

Chemically produced guanidinoacetic acid was dried after separation from the reaction mixture. The thus obtained product was used directly without any further processing in the testings.

2.3 Chemically produced GAA granulated

The procedure for preparing granulated chemically produced GAA corresponds to the general procedure for producing granulated GAA in paragraphs [0045] and [0046] of US 2010143703 A1.
Guanidinoacetic acid was placed in an intensive mixer at room temperature and homogenized for 1 minute. Subsequently, starch and water were added under slow stirring. Following, the mixer contents were stirred at 1500 to 2000 revolutions per minute, wherein a temperature increase took place. After 5 to 8 minutes granulating time, a granulate in the desired grain size range (32 to 2750 μm) was obtained. The granules were dried to a product temperature of 80 ° C

3. Determination of the particle size distribution

The particle size distribution was determined using a laser diffraction particle size analyzer (LA-950V2, Horiba). App. 300 mL iso-propanol was added, and the agitator was set to level 6. A blank measurement was started. A small amount of dry sample was added, and the measurement was started. For a second and a third measurement, ultrasonic was applied for 60 or 120 seconds at an ultrasonic setting of level 7. After each ultrasonic treatment the measurement was started, which resulted in three measurements: no ultrasonic treatment, ultrasonic treatment for 60 seconds, and ultrasonic treatment for 120 seconds. The evaluation of the measurement was done via the "Fraunhofer RT" method provided by the particle analyzer.
Table 1 summarizes the results of the determination of the particle size distribution, and Figures 1 and 2 illustrate said results.

4. Determination of the pourability

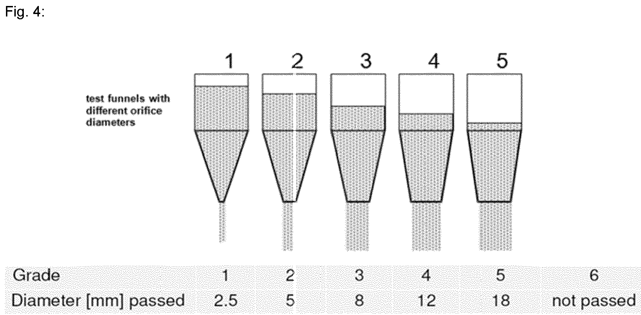

The pourability was determined using five different funnels, each with a different orifice diameter: The first funnel had an orifice diameter of 2.5 mm, which was the smallest orifice diameter. Product that passed orifice was graded with a pourability value of 1. Next were the second to fifth funnels, which had orifice diameters of 5 mm, 8 mm, 12 mm, and 15 mm. Product that passed any of these orifices was graded with a pourability value of from 2 to 5. Any product that did not pass the orifice of the fifth funnel was graded with a pourability value of 6. A pourability value of 5 or more indicates that the corresponding product is not suitable for automated, high output feed mills. Figure 4 illustrates the general procedure of this determination.

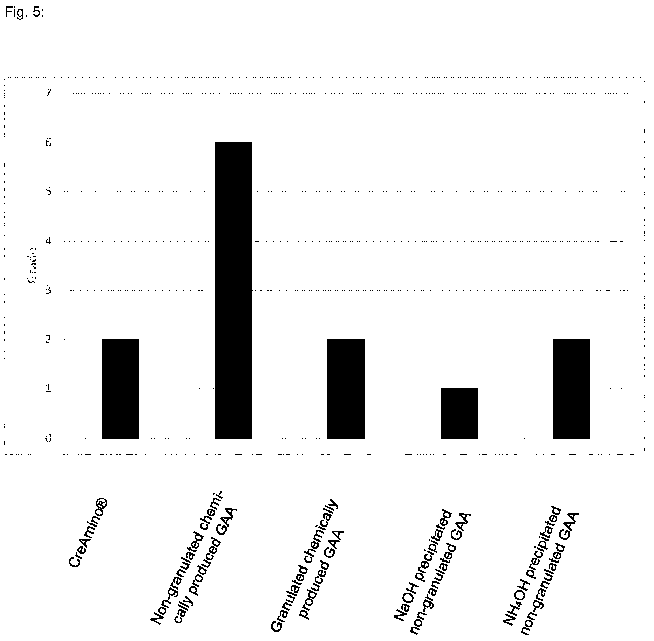

Table 1 and Figure 5 summarize the results of the determination of the pourability.

The results show that the guanidinoacetic acid particles according to the present invention and the guanidinoacetic acid particles obtained by the process according to the present invention always give improved flow characteristics, in particular an improved pourability, compared to commercially available guanidinoacetic acid (CreAmino®), and dried as well as granulated guanidinoacetic acid. These results are independent from the preceding production way for producing the guanidinoacetic acid used in the process according to the present invention. The process according to the present invention gave similar or even identical results for particles that were produced with guanidinoacetic acid that resulted from a chemical production and for particles that were produced with guanidinoacetic acid that resulted from a fermentative production.

Table 1: Overview of the results of the tested products

| Tested products | Sieve fraction [$\mu$m] | Funnel orifice [nm] | Grade |
|---|---|---|---|
| CreAmino® | > 200 | 5 | 3 |
| Chemically produced GAA dried | < 200 | > 24 | 6 |
| Chemically produced GAA granulated | > 200 | 5 | 3 |
| GAA ungranulated, NaOH precipitated | > 200 | 2.5 | 1 |
| GAA ungranulated, NH$_4$OH precipitated | > 200 | 5 | 2 |
| GAA ungranulated, fermentation | > 200 | 5 | 2 |

5. Determination of the angle of repose

Flow characteristics of powdered substances are determined by measuring the height of the constituted cone. For the experiment, a sieve with a mesh size, which the particles should pass unobstructed, was fixed in a distance of 60 mm above the top of a metal cylinder. The substance to be evaluated was gently rubbed through the sieve until a geometrically constant cone was formed on top of the metal cylinder. The angle of response was calculated by means of the formula:

$$Angle\ of\ repose\ [°] = tan^{-1}\frac{H}{r}$$

with

H = height of the cone in mm
r = radius of the metal cylinder in mm

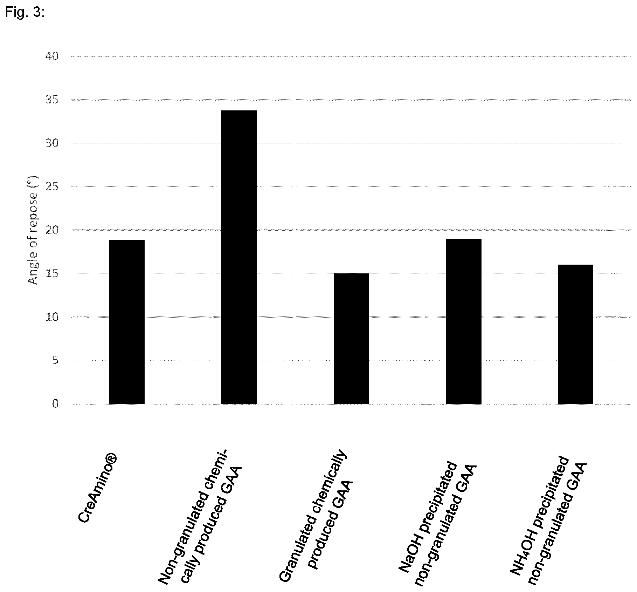

The results are summarized in Figure 3.

**Claims**

1. A process for the preparation of free-flowing particles comprising or consisting of an N-guanylamino acid, wherein the process comprises the steps of

    a) providing an N-guanylamino acid comprising solution or suspension,
    b) shifting the pH of the solution or suspension provided in step a) to a pH lower than the pKa value of the N-guanylamino acid, and
    c) shifting the pH of the solution or suspension obtained in step b) to a pH larger than the pKa value of the N-guanylamino acid and lower than the pKb value of the N-guanylamino acid, wherein the step c) further comprises the precipitation or crystallization of the particles comprising or consisting of the N-guanylamino acid.

    wherein the pH in each of the steps b) and c) is measured by means of a pH electrode.

2. The process according to claim 1, further comprising the steps

8

d) separating the precipitated or crystallized particles comprising or consisting of N-guanylamino acid from the solution or suspension obtained in step c), and
e) drying the particles separated in step d).

3. The process according to any of claims 1 to 2, wherein the solvent of the solution or suspension provided in step a) comprises or consists of water, an organic solvent, or a mixture thereof.

4. The process according to any of claims 1 to 3, wherein the pH value(s) set in step b) and/or c) is/are held for a time of up to 30 minutes.

5. The process according to any of claims 1 to 4, wherein the N-guanylamino acid is guanidinoacetic acid.

6. The process according to claim 5, wherein the pH of the solution or suspension obtained in step b) is lower than the pKa of guanidinoacetic acid.

7. The process according to claim 6, wherein the pH of the solution or suspension obtained in step b) is lower than 2.9.

8. The process according to any of claims 5 to 7, wherein the pH of the solution or suspension obtained in step c) is larger than the pKa of guanidinoacetic acid and lower than the pKb of guanidinoacetic acid.

9. The process according to claim 8, wherein the pH of the solution or suspension obtained in step c) is larger than 2.9 and lower than the pKb of guanidinoacetic acid.

10. The process according to claim 8, wherein the pH of the solution or suspension obtained in step c) is larger than 4 and lower than 10.91.

11. The process according to claim 8, wherein the pH of the solution or suspension obtained in step c) is between 5 and lower than 10.91.

12. The process according to any of claims 1 to 11, wherein the crystallization of guanidinoacetic acid is initiated at a pH or 5 or more.

13. The process according to any of claims 1 to 12, wherein the N-guanylamino acid comprising solution or the suspension provided in step a) or the N-guanylamino acid contained in said solution or suspension results from the chemical reaction of an amino acid with cyanamide.

14. The process according to any of claims 1 to 12, wherein the N-guanylamino acid comprising solution or the suspension provided in step a) or the N-guanylamino acid contained in said solution or suspension results from the fermentative production of the N-guanylamino acid.

**Patentansprüche**

1. Verfahren zur Herstellung von rieselfähigen Partikeln, die eine N-Guanylaminosäure umfassen oder daraus bestehen, wobei das Verfahren die folgenden Schritte umfasst:

   a) Bereitstellen einer N-Guanylaminosäure umfassenden Lösung oder Suspension,
   b) Verschieben des pH-Werts der in Schritt a) bereitgestellten Lösung oder Suspension auf einen pH-Wert, der kleiner als der pKa-Wert der N-Guanylaminosäure ist, und
   c) Verschieben des pH-Werts der in Schritt b) erhaltenen Lösung oder Suspension auf einen pH-Wert, der größer als der pKa-Wert der N-Guanylaminosäure und kleiner als der pKb-Wert der N-Guanylaminosäure ist, wobei der Schritt c) ferner die Fällung oder Kristallisation der Partikel, die die N-Guanylaminosäure umfassen oder daraus bestehen, umfasst,

   wobei der pH-Wert in jedem der Schritte b) und c) mit Hilfe einer pH-Elektrode gemessen wird.

2. Verfahren nach Anspruch 1, ferner umfassend die Schritte:

d) Abtrennen der ausgefällten oder kristallisierten Partikel, die N-Guanylaminosäure umfassen oder daraus bestehen, von der in Schritt c) erhaltenen Lösung oder Suspension und

e) Trocknen der in Schritt d) abgetrennten Partikel.

3. Verfahren nach einem der Ansprüche 1 bis 2, wobei das Lösungsmittel der in Schritt a) bereitgestellten Lösung oder Suspension Wasser, ein organisches Lösungsmittel oder eine Mischung davon umfasst oder daraus besteht.

4. Verfahren nach einem der Ansprüche 1 bis 3, wobei der/die in Schritt b) und/oder c) eingestellte(n) pH-Wert(e) für eine Zeit von bis zu 30 Minuten gehalten wird/werden.

5. Verfahren nach einem der Ansprüche 1 bis 4, wobei es sich bei der N-Guanylaminosäure um Guanidinoessigsäure handelt.

6. Verfahren nach Anspruch 5, wobei der pH-Wert der in Schritt b) erhaltenen Lösung oder Suspension kleiner als der pKa-Wert der Guanidinoessigsäure ist.

7. Verfahren nach Anspruch 6, wobei der pH-Wert der in Schritt b) erhaltenen Lösung oder Suspension kleiner als 2,9 ist.

8. Verfahren nach einem der Ansprüche 5 bis 7, wobei der pH-Wert der in Schritt c) erhaltenen Lösung oder Suspension größer als der pKa-Wert von Guanidinoessigsäure und kleiner als der pKb-Wert von Guanidinoessigsäure ist.

9. Verfahren nach Anspruch 8, wobei der pH-Wert der in Schritt c) erhaltenen Lösung oder Suspension größer als 2,9 und kleiner als der pKb-Wert von Guanidinoessigsäure ist.

10. Verfahren nach Anspruch 8, wobei der pH-Wert der in Schritt c) erhaltenen Lösung oder Suspension größer als 4 und kleiner als 10,91 ist.

11. Verfahren nach Anspruch 8, wobei der pH-Wert der in Schritt c) erhaltenen Lösung oder Suspension zwischen 5 und weniger als 10,91 liegt.

12. Verfahren nach einem der Ansprüche 1 bis 11, wobei die Kristallisation von Guanidinoessigsäure bei einem pH-Wert oder 5 oder mehr initiiert wird.

13. Verfahren nach einem der Ansprüche 1 bis 12, wobei die in Schritt a) bereitgestellte N-Guanylaminosäure umfassende Lösung oder Suspension oder die in der Lösung oder Suspension enthaltene N-Guanylaminosäure aus der chemischen Reaktion einer Aminosäure mit Cyanamid resultiert.

14. Verfahren nach einem der Ansprüche 1 bis 12, wobei die in Schritt a) bereitgestellte N-Guanylaminosäure umfassende Lösung oder Suspension oder die in der Lösung oder Suspension enthaltene N-Guanylaminosäure aus der fermentativen Produktion der N-Guanylaminosäure resultiert.

## Revendications

1. Procédé pour la préparation de particules à écoulement libre comprenant ou constituées par un N-guanylaminoacide, dans lequel le procédé comprend les étapes de

a) fourniture d'une solution ou suspension comprenant un N-guanylaminoacide,

b) déplacement du pH de la solution ou suspension fournie à l'étape a) jusqu'à un pH inférieur à la valeur de pKa du N-guanylaminoacide et

c) déplacement du pH de la solution ou suspension obtenue à l'étape b) jusqu'à un pH supérieur à la valeur de pKa du N-guanylaminoacide et inférieur à la valeur de pKb du N-guanylaminoacide, où l'étape c) comprend en outre la précipitation ou la cristallisation des particules comprenant ou constituées par le N-guanylaminoacide,

dans lequel le pH dans chacune des étapes b) et c) est mesuré au moyen d'une électrode de pH.

2. Procédé selon la revendication 1, comprenant en outre les étapes

d) séparation des particules précipitées ou cristallisées comprenant ou constituées de N-guanylaminoacide de la solution ou suspension obtenue à l'étape c), et

e) séchage des particules séparées à l'étape d).

3. Procédé selon l'une quelconque des revendications 1 à 2, dans lequel le solvant de la solution ou suspension fournie dans l'étape a) comprend ou est constitué d'eau, d'un solvant organique ou d'un mélange de ceux-ci.

4. Procédé selon l'une quelconque des revendications 1 à 3, dans lequel la ou les valeurs de pH fixées dans l'étape b) et/ou c) sont maintenues pendant une durée allant jusqu'à 30 minutes.

5. Procédé selon l'une quelconque des revendications 1 à 4, dans lequel le N-guanylaminoacide est l'acide guanidinoacétique.

6. Procédé selon la revendication 5, dans lequel le pH de la solution ou de la suspension obtenue dans l'étape b) est inférieur au pKa de l'acide guanidinoacétique.

7. Procédé selon la revendication 6, dans lequel le pH de la solution ou suspension obtenue à l'étape b) est inférieur à 2,9.

8. Procédé selon l'une quelconque des revendications 5 à 7, dans lequel le pH de la solution ou de la suspension obtenue dans l'étape c) est supérieur au pKa de l'acide guanidinoacétique et inférieur au pKb de l'acide guanidinoacétique.

9. Procédé selon la revendication 8, dans lequel le pH de la solution ou de la suspension obtenue dans l'étape c) est supérieur à 2,9 et inférieur au pKb de l'acide guanidinoacétique.

10. Procédé selon la revendication 8, dans lequel le pH de la solution ou suspension obtenue à l'étape c) est supérieur à 4 et inférieur à 10,91.

11. Procédé selon la revendication 8, dans lequel le pH de la solution ou suspension obtenue à l'étape c) est compris entre 5 et inférieur à 10,91.

12. Procédé selon l'une quelconque des revendications 1 à 11, dans lequel la cristallisation de l'acide guanidinoacétique est initiée à un pH de 5 ou plus.

13. Procédé selon l'une quelconque des revendications 1 à 12, dans lequel la solution comprenant le N-guanylaminoacide ou la suspension fournie dans l'étape a) ou le N-guanylaminoacide contenu dans ladite solution ou suspension résulte de la réaction chimique d'un acide aminé avec le cyanamide.

14. Procédé selon l'une quelconque des revendications 1 à 12, dans lequel la solution comprenant le N-guanylaminoacide ou la suspension fournie dans l'étape a) ou le N-guanylaminoacide contenu dans ladite solution ou suspension résulte de la production par fermentation du N-guanylaminoacide.

Fig. 1:

Fig. 2:

Fig. 3:

Fig. 4:

| Grade | | 1 | 2 | 3 | 4 | 5 | 6 |
|---|---|---|---|---|---|---|---|
| Diameter [mm] passed | | 2.5 | 5 | 8 | 12 | 18 | not passed |

Fig. 5:

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 102329250 A
- CN 101462983 A
- WO 2009012960 A
- CN 110663821 A
- US 2022388948 A1
- US 2010143703 A1

**Non-patent literature cited in the description**

- **M. STRECKER**. *compt. Rend.*, 1861, vol. 52, 1212
- Ber. Chem. Ges.. *Eur. J. Inorg. Chem.*, 1904, vol. 41, 4385